# EUROPEAN PATENT SPECIFICATION

(11) **EP 0 508 524 B1**
(45) Date of publication and mention of the grant of the patent: **19.06.1996**
(21) Application number: 92200911.3
(22) Date of filing: 31.03.1992
(51) Int. Cl.: A61K 33/30

(54) **Use of zinc ions for the treatment of periodontitis**
Verwendung von Zinkionen zur Behandlung der Periodontitis
Utilisation d'ions de zinc pour le traitement de la périodontite

(30) Priority: 12.04.1991 GB 9107833
(43) Date of publication of application: 14.10.1992
(73) Proprietor: UNILEVER N.V., NL-3013 AL Rotterdam (NL); UNILEVER PLC, London EC4P 4BQ (GB)
(72) Inventor: Hughes, Julia Mary, Unilever Research Colworth Lab, Bedford MK44 1LQ (GB); Hughes, Trevor C., Unilever Research Colworth Lab., Bedford MK44 1LQ (GB)
(74) Representative: van Gent, Jan Paulus

(56) References cited:
- WO-A-87/00051
- DE-A- 3 028 782
- GB-A- 2 159 412
- PATENT ABSTRACTS OF JAPAN, vol. 12, no. 297 (C-519)[3144], 12th August 1988; & JP-A-63 66 113
- INTERNATIONAL JOURNAL OF COSMETIC SCIENCE, vol. 13, no. 1, February 1991, pages 29-42; J. VERRAN "Dental plaque - associated infections and antibacterial oral hygiene products"
- JOURNAL OF PERIODONTOLOGY, vol. 61, no. 6, June 1990, pages 352-358; D.S. HARPER et al.: "Clinical efficacy of a dentifrice and oral rinse containing sanguinaria extract and zinc chloride during 6 months of use"
- JOURNAL OF CLINICAL PERIODONTOLOGY, vol. 17, no. 8, September 1990, pages 570- 574; C.L. JONES et al.: "Microbiological and clinical effects of a dentifrice containing zinc citrate and Triclosan in the human experimental gingivitis model"

## Description

The present invention relates, to the inhibition and/or reduction of periodontitis by the use of a medicament that comprises a zinc-ion containing compound.

Periodontitis is a general term describing specific diseases affecting the gingiva and the supporting connective tissue and alveolar bone which anchor the teeth in the jaws. Periodontitis causes loss of connective tissue, formation of periodontal pockets, and resorption of alveolar bone and may lead to a loosening of the teeth, and ultimately to the loss of teeth.

Periodontal disease is mainly caused by specific anaerobic bacteria in the periodontal pockets. The destruction of the periodontal tissue is primarily caused by the indirect effects mediated by the host's defensive reactions to the bacteria. Bacterial metabolites induce leucocyte chemotaxis which results in the inflammatory cells accumulating at the site of the bacterial challenge. Furthermore, bacterial metabolites induce the production of inflammatory mediators by leucocytic cells, in particular monocytes and macrophages. Amongst these are local disease mediators such as metabolites of arachidonic acid, e.g. leukotrienes, prostaglandins and thromboxanes. Additionally, the loss of alveolar bone may be directly induced by pathogenic metabolites of bacteria, in particular proteolytic enzymes, lipopolysaccharides and capsular proteins. Prostaglandins have been found to be particularly important in the metabolism and destruction of tissue and alveolar bone. Indeed, the levels of prostaglandins in the periodontal tissues and crevicular fluid has been found to be closely associated with the loss of alveolar bone in the periodontium; i.e. patients with periodontal breakdown show an elevated prostaglandin E₂ (PGE₂) level both in the gingival tissue as well as in the crevicular fluid. Inhibiting the formation of prostaglandins thus reduces alveolar bone loss. Indeed certain prostaglandin formation inhibitors, particularly non steroidal anti-inflammatory drugs such as indomethacin and flurbiprofen, have been found to be able to reduce the resorption of alveolar bone in animal models and human studies.

There are literature data that zinc salts can stimulate the growth of bone in vivo and in vitro. Yamaguchi et al in J. Pharmacobiodyn. 5, 619-626, 1982 and J. Bone Miner, Metab. 2, 186, 1984 teach that administration of zinc sulphate over 3 days to weanling rats causes a statistically significant increase in bone growth as measured by mg calcium per g of bone ash. This increase in bone formation is accompanied by other biochemical changes associated with a bone growth (e.g. elevated alkaline phosphatase levels) and with a statistically significant increase in bone zinc levels. Furthermore Yamaguchi, M. et al. also disclose in Biochemical Pharmacology 36 (22), 4007-4012, 1987 that zinc sulphate (10⁻⁴ to 10⁻⁶ moles per litre) can stimulate bone growth in 3w old rats bones placed into tissue culture for 2 or 3 days, as measured by a significant increase in calcium content. This observation is also accompanied by statistically significant increases in bone associated zinc. Yamaguchi has reviewed the role of zinc as a stimulating factor in bone formation in an article in Eisei Kagaku 36(2), 85-99, 1990.

There are also numerous if largely unsupported remarks to the wound healing benefits derived from zinc and zinc is purported to concentrate at sites of early wound repair (Savlov, E.D. et al. Journal of Surg. Research 2, 209-212, 1962). However none of these references or articles teach that topical zinc administration can offer periodontitis benefits or reduce the loss of alveolar bone.

We have now found that zinc-ion containing compounds can significantly reduce alveolar bone resorption. While the exact mechanism through which this effect is obtained is not known to us, it is believed that the zinc ions may operate via either (i) an anti-inflammatory route, inhibiting the biological effects of, for example PGE₂ (Suzuki, Y. et al., Toxicology 62(1), 1990, p. 27-34) or interleukin-1-beta synthesis or (ii) by directly inhibiting bone resorption. The zinc-ion containing compounds may affect the function of specialised bone cells (principally osteoblasts and osteoclasts) on the bone surface which regulate bone growth and bone loss respectively or by direct effects on the mineralised bone surface. The zinc-ion may reduce breakdown of connective tissue/ligaments and/or increase synthesis of ligaments.

Consequently, the present invention relates to the use of zinc-ion containing compounds in the manufacture of a medicament for inhibiting alveolar bone resorption. It relates especially to the use of zinc-ion containing compounds in the manufacture of a medicament for reducing periodontitis.

Zinc-ion containing compounds are well-known anti-bacterial agents and are used in oral compositions to reduce or inhibit the growth of dental plaque. Their use to inhibit alveolar bone resorption or periodontitis has not been indicated in the prior art as far as we know. In the International Journal of Cosmetic Science, Vol. 13, (1991), pages 29-42 it is stated that prevention of periodontitis can be achieved by adequate plaque removal, but no suggestion is made of any particularly suitable agent to achieve such prevention of periodontitis.

Since many zinc-ion containing compounds also have anti-bacterial activity, they not only modulate the host response system by inhibiting e.g. bone resorption and prostaglandin formation, but also reduce the microbial challenge, thus having a highly desirable combined, dual effect to reduce periodontitis.

Suitable examples of zinc-ion containing compounds are zinc salts with organic or inorganic counterions, such as zinc salts of the following organic and inorganic anions: acetate, benzoate, borate, bromide, carbonate, glycinate, glutamate, citrate, chloride, glycerophosphate, hexafluorosilicate, dl-lactate (trihydrate),nitrate, phenolsulfonate, silicate, alkanoates having 8 to 18 carbon atoms, such as zinc stearate, salicylate, stannate, sulfate, tannate, titanate, tetrafluoroborate, oxide, peroxide, tartrate, etc. Mixtures of zinc compounds may also be used. Further suitable examples are alkalimetal zincosilicates, hydrotalcites and analogs thereof, zinc-ion containing minerals like basic zinc chloride, zinc hydroxy apatite and so on. Still further suitable examples are the zinc salt of 2-(2,6-dichloroaniline) phenylacetic acid, the zinc salt of indomethacin, the zinc salt of 2-(6-chlorocarbazol-2-yl) proprionate, β-alanyl-L-histidinato zinc, and zinc monoglycerolate. Zinc citrate and zinc glycinate are preferred zinc-ion containing compound.

By the term "zinc-ion" as used herein, we are referring to the Zn portion of a zinc compound capable of either dissociating into zinc ions at a temperature of about 37° C, or the zinc portion of a zinc compound capable of biological utilisation in humans, as well as to hydrated or otherwise substituted zinc ions formed in aqueous medium such as a mouthwash or oral salivary secretions.

In general, the proportion of zinc compound will be kept at a minimum effective level to avoid undue astringency. The level can be higher for the more insoluble compounds. The zinc compounds referred to as "slightly soluble" have sufficient, although in some instances almost negligible, solubility in water to furnish zinc ions. However, even "slightly soluble" zinc salts can still deliver biologically available zinc by either slow dissolution on tissue surfaces or by mucosal or gastrointestinal absorption.

The zinc compounds may be present in the composition in amounts sufficient to furnish about 0.05% to about 10%.

Slightly soluble zinc compounds such as zinc citrate or zinc oxide, may be present within a range of proportions equivalent to about 0.05% to about 10% Zn ion, preferably about 0.2% to about 1.5%, while a soluble zinc compound, for example zinc chloride, will usually be present in lesser proportions, for example in amounts equivalent to about 0.05% to about 4.0% Zn ion, preferably about 0.1% to about 1.0%.

The zinc-ion containing compound-containing medicament of the present invention can be manufactured in any form, suitable for administering the medicament to achieve the reduction or prevention of periodontitis. Such forms are tablets, capsules, pills, powders, granules, solutions, suspensions, salves, gels, pastes etc. Suitable forms for oral administration are toothpastes, mouthwashes, gels, sols and the like. Also it is possible to formulate the medicament in forms, suitable for topical and buccal administration, e.g. for dosing in periodontal pockets and/or tissues by special applications, such as gels, irrigator fluids, flosses, chewing gum, lozenges, fibres (hollow and monolytic), adhesive strips, tooth picks and the like.

The medicament furthermore may comprise further, conventional ingredients, such as pharmaceutically acceptable carriers including starch, sucrose, polyols, surfactants, water or water/alcohol systems etc. When formulated into a dentifrice, such formulation may contain all the usual dentifrice ingredients. Thus, they may comprise particulate abrasive materials such as silicas, aluminas, calcium carbonates, dicalciumphosphates, hydroxyapatites, trimetaphosphates, insoluble hexametaphosphates and so on, usually in amounts between 5 and 60% by weight.

Furthermore, they may comprise humectants such as glycerol, sorbitol, propyleneglycol, lactitol and so on.

Surface-active agents may also be included such as anionic, nonionic, amphoteric and zwitterionic synthetic detergents. Examples thereof are sodiumlaurylsulphate, sodium dodecylbenzenesulphonate, sodium mono- and dioctyl-phosphate, sodiumlauroylsarcosinate.

Binders and thickeners such as sodium carboxymethylcellulose, xanthan gum, gum arabic etc. may also be included, as well as synthetic polymers such as polyacrylates, copolymers of polyvinylmethylether with maleic anhydride. Flavours such as peppermint and spearmint oils may also be included, as well as preservatives, opacifying agents, colouring agents, pH-adjusting agents, sweetening agents and so on.

Anti-plaque agents may also be included such as chlorhexidine, copper- and stannous salts, Triclosan, sanguinarine extract, metronidazole; furthermore anti-inflammatory agents such as ibuprofen, flurbiprofen, aspirin, indomethacin, piroxycam, naproxen etc. may also be included. Anti-caries agents such as sodium- and stannous fluoride, aminefluorides, monosodiumfluorophosphate, casein and casein digests may also be included. Vitamins such as Vitamin C, plant extracts, potassium salts such as potassium citrate and potassium nitrate can also be included.

Anti-bacterial agents such as quaternary ammonium compounds e.g. cetylpyridinium chloride; bis-guanides such as chlorhexidine digluconate, hexetidine, octenidine, alexidine may also be included.

Other anti-plaque agents include enzymes such as dextranase and/or mutanase, amyloglucosidase, glucose-oxidase with lactic oxidase, neuro amidases, hydrogenperoxide generating compounds such as potassiumperoxydiphospate. Targeted anti-body systems, containing a therapeutic agent directed against plaque-causing bacteria and an anti-target anti-body and a further anti-body, linking the therapeutic agent to the anti-target anti-body may also be used.

The invention will be further illustrated by way of Example.

### Example 1

A silk ligature was tied around each second maxillary molar of Osborne-Mendel strain rats at 11 weeks of age on a level with the gingival margin. Care was taken to ensure that the knot in the ligature was always placed at the same point i.e. on the palatal aspect adjacent to the distal root.

### Treatments

1) Fifty animals from 5 litters were allocated as appropriate to allow for variations in responses between litters and sexes to 3 treatment groups and treated with either mouthwash a) or b) or with distilled water c)
Complete formulations for mouthwashes a) and b) are given below.

| Formulations of test mouth rinses | | |
|---|---|---|
| Ingredients | Placebo + zinc | Placebo |
| | a % | b % |
| Ethanol | 15.0 | 15.0 |
| Zinc phenol sulphonate | 0.7 | - |
| Polyethylene glycol 400 | 40.0 | 40.0 |
| Ethoxylated hydrogenated castor oil | 0.5 | 0.5 |
| Flavour | 0.2 | 0.2 |
| Colouring agent | 0.00025 | 0.00025 |
| Water to 100% | | |

Treatments were applied twice daily (weekends excepted) to the gingival sulcus, by syringe at a dose rate of 0.2ml. Treatment commenced after ligation and continued for a period of 4 weeks.
2) One hundred and thirty two animals from 16 litters were allocated as described above to 4 treatment groups, and treated with toothpastes a, b, or c, or with distilled water.

Complete formulations for toothpastes a), b) and c) are given below.

| Formulations of toothpastes | | | |
|---|---|---|---|
| Ingredients | 1.0% Zinc citrate a | 0.5% Zinc citrate b | Placebo c |
| Zinc citrate trihydrate | 1.000 | 0.500 | 0.000 |
| Alumina | 50.000 | 50.000 | 50.000 |
| Sodium carboxymethylcellulose | 0.800 | 0.800 | 0.800 |
| Sodium laurylsulphate | 1.875 | 1.875 | 1.875 |
| Sodium dodecylbenzenesulphonate | 0.625 | 0.625 | 0.625 |
| Sorbitol Syrup (70% w/w) | 27.000 | 27.000 | 27.000 |
| Saccharin | 0.180 | 0.180 | 0.180 |
| Flavour | 1.200 | 1.200 | 1.200 |
| Sodium monofluorophosphate (MFP) | 0.850 | 0.850 | 0.850 |
| Formalin | 0.040 | 0.040 | 0.040 |
| Water to 100% | | | |

### Measurement of vertical loss of alveolar bone

After 4 weeks of treatment animals were sacrificed and the maxillae were removed. Two methods were used for evaluation of vertical loss of bone.
1. Scanning electron microscopy
2. Conventional histology

1. Scanning electron microscopy
   This method was used for evaluation of both left and right maxillae of animals from mouthwash experimental group and for right maxillae of toothpaste experimental group.
   After preparation specimens were examined under the scanning electron microscope (Jeol T100) and maxillae were photographed at low magnification (X20) at right angles to the vertical axis. Vertical loss of bone was determined by measuring the maximum distance between the cemento-enamel junction (CEJ) and the jaw bone in an experimental specimen, minus maximum depth of a non ligated control. All measurements were carried out using an image analysis system (Graphic Information Systems Limited).
2. Conventional histology
   This method was used for evaluation of left maxillae from toothpaste experimental group.
   After excision maxillae were fixed in ½ strength Karnovsky (Karnovsky 1965) for 48 hours at 4°C they were then transferred to 10% EDTA, pH 7.4 for 6 weeks, the end point of decalcification being determined radiographically. After decalcification tissues were embedded in paraffin. Serial sections were cut at 5µ and stained by haematoxylin and eosin. Ten serial sections were mounted per slide. Loss of alveolar bone was determined by measuring the straight line distance from the cemento-enamel junction to the alveolar bone crest (mean of 10 sections). Measurements were carried out using an image analysis system (Graphic Information Systems limited).

### Statistical Methods

Vertical loss of alveolar bone from left and right maxillae was used to calculate a mean figure of loss of alveolar bone per animal. These data were then analysed by 2 way analysis of variance followed by Duncan's multiple range test (Duncan 1955).

### Results

Results for vertical loss of alveolar bone following treatment with mouthwash and toothpaste formulations are given in Tables 1 and 2. Mouthwash formulation a) containing zinc significantly (p ≦ 0.05) reduced loss of bone when compared to treatment with c) (Table 1). Numerically, the zinc containing formulation was also superior to its true placebo b) but the difference was not statistically significant (Table 1). Treatment with a placebo toothpaste containing MFP (0.85%) also reduced loss of alveolar bone significantly (p ≦ 0.05) compared to treatment with water (Table 2). Inclusion of zinc citrate in the toothpaste at 0.5 or 1.0% reduced loss of alveolar bone further (Table 2). In the case of zinc citrate (1.0%) the reduction was statistically significant (p ≦ 0.05) compared to the placebo paste.

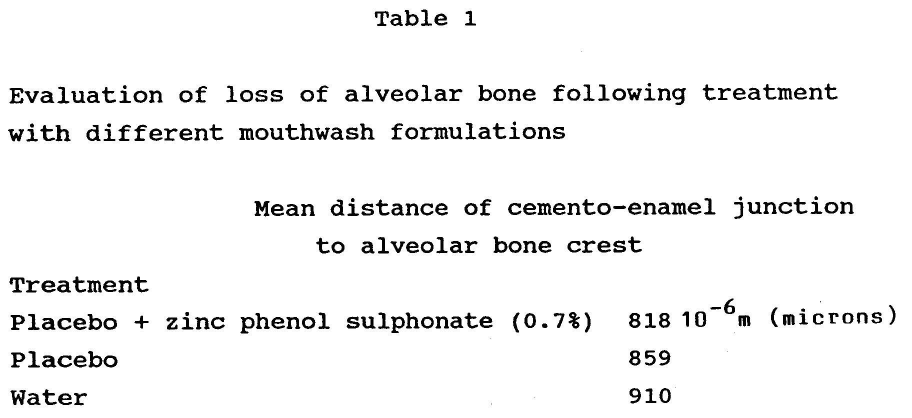

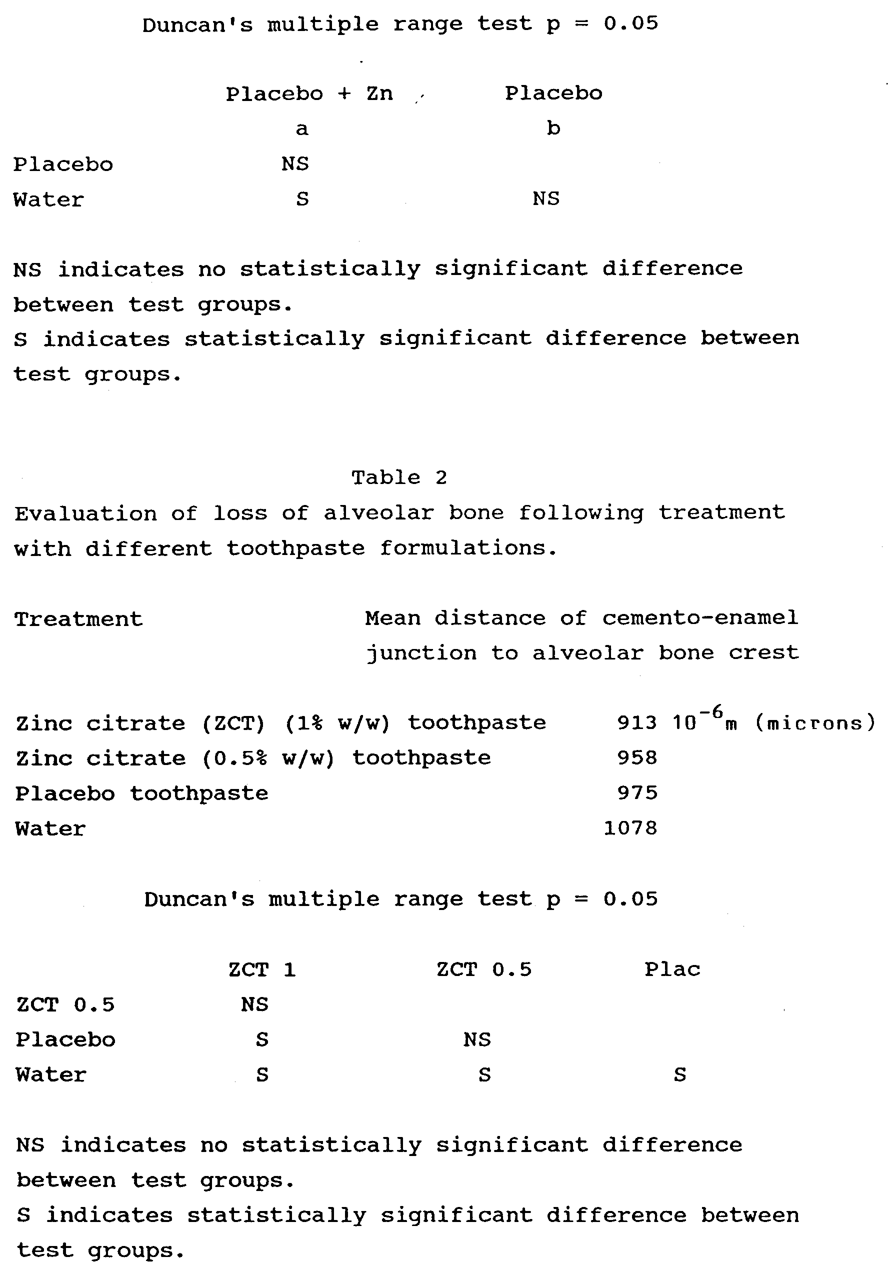

### Example 2

### EFFECT OF ZINC SULPHATE ON BONE LOSS IN VITRO

### Method

The experimental method used is fully described in Meghi,S et al. Brit. J. Cancer (1988) 58, pp 17-21. Neonatal mouse calvaria were isolated with minimum trauma and maintained in a nutrient medium appropriate for cell culture (DMEM + 15% horse serum) at 27°C in 100% humidity. The pH was maintained in an atmosphere of 5% CO₂. After 24h the medium was discarded, the calvaria were divided into control and test groups, and the medium was replaced with fresh medium containing the materials under test. Prostaglandin E₂ (PGE₂, 10⁻⁶ M) was added with or without zinc sulphate as appropriate to stimulate the release of calcium ions (i.e. bone resorption) from the calvaria. After two days further culture in the test media the release of calcium into the medium from the calvaria was determined by atomic absorption spectroscopy. Calcium release is a measure of bone resorption (i.e. degradation of bone) regulated by the specialised cells (osteoblasts and osteoclasts) on the bone surface. Net calcium release was obtained by subtracting the basal medium calcium level.

| Additions | Net Calcium Release |
|---|---|
| | (mg/dl) (n) |
| None (negative control) | 0.0 (7) |
| + PGE₂ (positive control) | 1.9 (8) |
| + PGE₂ and 10⁻⁶M zinc sulphate | 1.8 (3) |
| + PGE₂ and 10⁻⁵M zinc sulphate | 0.7 (5)* |
| + PGE₂ and 10⁻⁴M zinc sulphate | 0.5 (3)** |

| | |
|---|---|
| (n) number of measurements | |
| * Significant reduction compared to positive control. (*p< 0.02), | |
| (**p< 0.01). | |

### Example 3

### Effect of zinc citrate on bone loss in vitro

### Method

The experimental method was the same as in example 2 except that zinc citrate replaced zinc sulphate.

| Additions | Net Calcium Release |
|---|---|
| | (mg/dl) (n) |
| None (negative control) | 0.3 (9) |
| + PGE₂ (positive control) | 1.8 (7) |
| + PGE₂ and 1x10⁻⁵M zinc citrate | 1.6 (5) |
| + PGE₂ and 2x10⁻⁵M zinc citrate | 1.5 (5) |
| + PGE₂ and 5x10⁻⁵M zinc citrate | 1.1 (5) |
| + PGE₂ and 1x10⁻⁴M zinc citrate | 0.9 (4)* |
| + PGE₂ and 3x10⁻⁴M zinc citrate | 0.4 (4)** |

| | |
|---|---|
| (n) number of incubations | |
| * significant reduction compared to the positive control (*p <0.05; | |
| **p < 0.01) | |

The data show that zinc sulphate and zinc citrate, in a dose response manner are able to inhibit the release of calcium from cultured mouse bone in vitro. The levels of zinc sulphate and zinc citrate required to achieve this are moderately low and non-toxic. PGE₂ is known to be a principal mediator of alveolar bone loss in periodontitis patients.

### Example 4

### The Delivery of Topically Applied Soluble Zinc to Alveolar Bone

### Method

Osborne-Mendel rats aged 21-23 days were treated with 5% aqueous zinc citrate solution or a toothpaste without zinc compounds. Treatments of 0.1g were applied twice per day to the molar teeth. The solution was given by pipette and the paste by soft brush. The zinc-treated and control groups contained 19 animals each and were balanced for sex and litter. The animals were sacrificed after 3 weeks treatment and the lower jaws removed, defleshed, rinsed with water and 70% alcohol and air dried. After further treatment in a pressure cooker for 45 min at 15x4.448 N(lb) pressure, the molar teeth were removed from the jaws. The zinc content of the alveolar bone from the molar region above the level of the incisal root was determined by drying at 100°C, dissolving in concentrated nitric acid and using inductively coupled plasma atomic absorption.

### Results

The zinc content of the alveolar bone from the zinc-treated animals was 310µg/g dry tissue ± 25 (SD) compared with 289µg/g ± 22 for control animals. The difference was statistically significant (p = 0.009) by a t-test. The calcium content was also increased significantly (p = 0.048) by the zinc-treatment from 24.0% ± 0.9 to 24.7% ± 0.9.

The results showed that oral topical application of zinc citrate solution delivered zinc to alveolar bone and increased the calcium content of the bone.

The above examples show that: zinc-ion containing compounds can reduce bone loss in vitro (examples 2 and 3), be delivered to jaw bone and affect jaw bone composition in vivo following topical administration (example 4) and can reduce loss of alveolar bone (i.e. periodontitis) by topical administration either as a mouthwash or toothpaste in an in vivo model (example 1).

## Claims

1. Use of a zinc-ion containing compound in the manufacture of a medicament for inhibiting bone resorption of alveolar and loss of periodontal ligament.

2. Use of a zinc-ion containing compound in the manufacture of a medicament for reducing periodontitis.

3. Use according to claim 1 or 2, in which the zinc-ion containing compound is zinc citrate.

4. Use according to claim 1 or 2, in which the zinc-ion containing compound is zinc glycinate.

5. Use according to any preceding claim, in which the medicament is suitable for oral administration.

6. Use according to claim 5, in which the medicament is in the form of a toothpaste or mouthwash.

## Patentansprüche

1. Verwendung einer Zinkionen enthaltenden Verbindung bei der Herstellung eines Medikaments zur Hemmung einer Alveolarknochenresorption und eines Verlusts von periodontalem Ligament.

2. Verwendung einer Zinkionen enthaltenden Verbindung bei der Herstellung eines Medikaments zur Verringerung von Periodontitis.

3. Verwendung nach Anspruch 1 oder 2, bei der die Zinkionen enthaltende Verbindung Zinkcitrat ist.

4. Verwendung nach Anspruch 1 oder 2, bei der die Zinkionen enthaltende Verbindung Zinkglycinat ist.

5. Verwendung nach einem der vorhergehenden Ansprüche, bei der sich das Medikament zur oralen Verabreichung eignet.

6. Verwendung nach Anspruch 5, bei der das Medikament in Form einer Zahncreme oder eines Mundwassers vorliegt.

## Revendications

1. Utilisation d'un composé contenant un ion zinc dans la fabrication d'un médicament pour inhiber la résorption de l'os alvéolaire et la perte du ligament périodontique.

2. Utilisation d'un composé contenant un on zinc dans la fabrication d'un médicament pour réduire la périodontite.

3. Utilisation selon la revendication 1 ou 2, dans laquelle le composé contenant un ion zinc est le citrate de zinc.

4. Utilisation selon la revendication 1 ou 2, dans laquelle le composé contenant un ion zinc est le glycinate de zinc.

5. Utilisation selon l'une quelconque des revendications précédentes, dans laquelle le médicament est approprié pour l'administration orale.

6. Utilisation selon la revendication 5, dans laquelle le médicament est sous forme d'un dentifrice ou d'un bain de bouche.
